# EUROPEAN PATENT SPECIFICATION

(11) **EP 3 198 275 B1**
(45) Date of publication and mention of the grant of the patent: **10.03.2021**
(21) Application number: 15843602.2
(22) Date of filing: 24.09.2015
(51) Int. Cl.: C12Q 1/22

(54) **STERILIZATION COMPOSITIONS AND METHODS**
STERILISATIONSZUSAMMENSETZUNGEN UND VERFAHREN
COMPOSITIONS STERILIZATION ET MÉTHODES

(30) Priority: 25.09.2014 US 201462055346 P
(43) Date of publication of application: 02.08.2017
(73) Proprietor: SPS Medical Supply Corp., Rush, NY 14543 (US)
(72) Inventor: DOYLE, Shawn, Cuba, NY 14727 (US); NOLAN, Mike, Rush, NY 14543 (US)
(74) Representative: Huygens, Arthur Victor
(86) International application number: PCT/US2015/051939
(87) International publication number: WO 2016/049311

(56) References cited:
- WO-A1-99/53019
- US-A- 5 739 004
- US-A- 5 739 004
- US-A1- 2003 215 923
- FURUKAWA S ET AL: "Formation of the spore clumps during heat treatment increases the heat resistance of bacterial spores", INTERNATIONAL JOURNAL OF FOOD MICROBIOLOGY, ELSEVIER BV, NL, vol. 102, no. 1, 25 June 2005 (2005-06-25) , pages 107-111, XP027663005, ISSN: 0168-1605 [retrieved on 2005-06-25]

## Description

### BACKGROUND

Sterilization is a process conducted in a specially designed chamber or sterilizer that results in a complete eradication of all viable micro-organisms. Sterilization techniques have evolved over time from the traditional methods employing saturated steam at elevated temperature and ethylene oxide gases to more modern techniques such as those employing liquid, vapor, and plasma. The effectiveness of the applied sterilization process must be evaluated regardless of the technique utilized especially when sterilization of instruments and devices invasive to the human body are concerned.

Biological indicators are devices that are used to test the efficacy of sterilization chambers such as those employed in hospitals for sterilizing medical and surgical instruments. Typically the biological indicator includes a source of microorganisms, a culture medium, and a visible detector to indicate the presence or absence of viable microorganisms.

In practice, the source of microorganisms, typically a carrier, such as, an absorbent paper strip that has been impregnated with a pre-determined concentration of live microorganisms, is subjected to the sterilization process. Thereafter, the microorganism impregnated strip is placed in a sterile culture medium and incubated for a predetermined time at an appropriate temperature. At the end of the incubation period, the detector or a visual inspection can be used to determine whether any microorganisms survived the sterilization process.

A typical biological indicator being used today is a self-contained biological indicator (SCBI) which contains all of the necessary features of the biological indicator-microorganisms, culture medium, and a detector.

One features of the SCBI is that upon activation, the possibility of exogenous contamination is reduced to a minimum thereby reducing a false positive reading to a minimal statistical probability. However, the same feature can have an adverse effect if residue of the hydrogen peroxide sterilizing agent is trapped inside of the SCBI micro-environment. This sterilizing agent residue can have an inhibitory effect on microorganism growth and give a false negative test, which can result in potentially contaminated items present among those items sterilized. The sterilizing agent residue problem occurs in both hydrogen peroxide and hydrogen peroxide/peracetic acid sterilizers as well as in hydrogen peroxide vapor sterilizers in the form of plasma, vapor and condensate.

During the sterilization cycle, hydrogen peroxide can be absorbed by the paper strip or spore dot that contains the viable microorganisms. At the end of the sterilization cycle, viable microorganisms may remain indicating the sterilization technique was ineffective.

However, the surviving microorganisms can be killed by the subsequent release of the absorbed hydrogen peroxide sterilant. Thus, when the SCBI is activated by adding the growth promoting media, there would be no growth of microorganisms, giving a false negative result.

The problem of neutralizing hydrogen peroxide and other oxidant residues has been addressed in the prior art. For example, U.S. Pat. No. 4,829,001 discloses a method for disinfecting a medical device comprising steps of immersing the device in hydrogen peroxide solution for a time sufficient to disinfect the device and decomposing any residual hydrogen peroxide by use of a catalytically effective amount of catalase or peroxidase mobilized on a composite article. Enzymatic decomposition leads to liberation of oxygen and creation of water or in the case of peracetic acid, acetic acid which can also be neutralized. Thus, if viable microorganisms survive the sterilization procedure they will thrive and reproduce in a growth promoting medium containing catalase when incubated and will be detected.

Moreover, the microorganisms, especially if in spore form, tend to clump together during inoculation, making it more difficult for the sterilization process to penetrate all of the spores within a clump. The outer spores are in a clump, and therefore prevent the inner spores from being properly exposed to the sterilant, which results in false sterilization positives. The microorganisms can also be dehydrated, which impedes penetration of the sterilant. When the sterilant is impeded from penetrating the microorganisms (e.g., spores), this can also result in false sterilization positives.

Commercially available SCBI's can be utilized for monitoring plasma/vapor hydrogen peroxide and hydrogen peroxide/peracetic acid sterilization cycles. However, for the reasons indicated above, they may potentially indicate false completion of the sterilization cycle after incubation.

Thus, there is a need for an improved and more accurate SCBI, which reduces inaccurate results of the sterilization process.

U.S. Pat. No. 5,739,004 discloses a biological indicator which utilizes immobilization to increase the thermostability of the biomaterial (e.g., microorganism or enzyme) contained within the indicator. The patent does not disclose or make obvious that the carrier comprises a glass and the glass comprises a fiber having as diameter between 1 to 500 microns.

### SUMMARY

A biological indicator for measuring effectiveness of a sterilization procedure is provided, which reduces inaccurate results in the sterilization process.

Sterilization compositions and methods are provided that comprise at least one of a humectant, anti-agglomerating agent or surfactant disposed onto a carrier of a biological indicator, which decreases the clumping of spores and increases spore hydration, thereby increasing sterilization penetration into the spores to reduce false positive sterilization results. The biological indicator for measuring effectiveness of a sterilization procedure comprising: a carrier having a microorganism and having from 0.5 w/w% glycerol to 5 w/w% glycerol, the carrier comprising a paper or a glass wherein the glass comprises a fiber having a diameter between 1 to 500 microns.
In various embodiments, the carrier comprises (i) 0.5 w/w% glycerol and 99.5 w/w% sterile fluid; (ii) 1.0 w/w% glycerol and 99.0 w/w% sterile fluid; (iii) 2.0 w/w% glycerol and 98.0 w/w% sterile fluid; or (iv) 5.0 w/w% glycerol and 95.0 w/w% sterile fluid.
In an embodiment, the microorganism comprises spores comprising Bacillus, Clostridium, Neurospora, Candida, Cryptosporidium, Geobacillus stearothermophilus and/or Bacillus atrophaeus.
In another embodiment, the biological indicator further comprises (i) a container comprising growth media wherein the carrier is separated from the growth media; (ii) the container comprises a member configured to move the carrier from a first position separated from the growth media to a second position to contact the growth media; or (iii) the media is disposed within a crushable vial.
In still another embodiment, the carrier comprises a deformable container having a modulus of elasticity from 1 x 10⁻² to 6 x 10⁵ dyn/cm².

Also provided is a method for determining effectiveness of a sterilization procedure with a biological indicator, the method comprising: subjecting the biological indicator to a sterilization cycle, the biological indicator comprising a deformable container having a crushable vial disposed therein comprising growth media and a carrier separated from the crushable vial but within the deformable container, wherein the carrier having from 0.5 w/w% glycerol to 5 w/w% glycerol, the carrier comprising a glass wherein the glass comprising a fiber having a diameter between 1 to 500 microns and spores; activating the biological indicator by applying pressure to said deformable container to crush the vial so as to allow the growth media and the spores of the carrier to come into contact with each other; and incubating the biological indicator for a predetermined period of time to permit any growth of the spores surviving said sterilization cycle to grow in the growth media, wherein the growth provides a determination of the effectiveness of the sterilization procedure.
In an embodiment, (i) the sterilization procedure comprises contacting the spores with hydrogen peroxide sterilization, steam sterilization and ethylene oxide sterilization; or (ii) the sterilization procedure comprises contacting the spores with hydrogen peroxide or peracetic acid.
In another embodiment of the method, the biological indicator further comprises a vented cap which allows a sterilant from the sterilization procedure to enter the deformable container and contact the spores on the carrier during the sterilization cycle.
In a further embodiment, a positive indication of spore growth is shown via a pH indicator and the pH indicator changes color in the growth media.
In still another embodiment of the method, the carrier comprises a deformable container having a modulus of elasticity from 1 x 10⁻² to 6 x 10⁵ dyn/cm².

While multiple embodiments are disclosed, still other embodiments of the present disclosure will become apparent to those skilled in the art from the following detailed description, which shows and describes illustrative embodiments of the disclosure. As will be realized, the various embodiments of the present disclosure are capable of modifications in various obvious aspects. Accordingly, the detailed description is to be regarded as illustrative in nature and not restrictive, the scope of the invention being indicated by the appended claims.

### BRIEF DESCRIPTION OF DRAWINGS

In part, other aspects, features, benefits and advantages of the embodiments will be apparent with regard to the following description, appended claims and accompanying drawings where:
FIG. 1 is a front view of one embodiment of the components of the biological indicator.
FIG. 2 is a front view of the biological indicator carrier having an inoculum of microorganisms on it and at least one of a humectant, anti-agglomerating agent or surfactant disposed thereon.
FIG. 3 is a front view of one embodiment of the components of the biological indicator where the carrier is in a first position before or during the sterilization process so that the growth media is not in contact with the carrier when the inoculum of microorganisms on the carrier comes in contact with the sterilant.
FIG. 4 is a front view of one embodiment of the components of the biological indicator where the carrier is moved into a second position just after sterilization, where the inoculum of microorganisms on the carrier come in contact with the growth media just after the sterilization process ends.
FIG. 5 is a break-away top view of one embodiment of the components of the biological indicator.
FIG. 6 is a break-away top view of one embodiment of the components of the biological indicator showing a cap filter and a label.
FIG. 7 is a top view of one embodiment of the components of the biological indicator.

It is to be understood that the figures are not drawn to scale. Further, the relation between objects in a figure may not be to scale, and may in fact have a reverse relationship as to size. The figures are intended to bring understanding and clarity to the structure of each object shown, and thus, some features may be exaggerated in order to illustrate a specific feature of a structure.

### DETAILED DESCRIPTION

For the purposes of this specification, unless otherwise indicated, all numbers expressing quantities of ingredients, percentages or proportions of materials, reaction conditions, and other numerical values used in the specification, are to be understood as being modified in all instances by the term "about." Accordingly, unless indicated to the contrary, the numerical parameters set forth in the following specification are approximations that may vary depending upon the desired properties sought to be obtained by the embodiments of the present disclosure. At the very least, each numerical parameter should at least be construed in light of the number of reported significant digits and by applying ordinary rounding techniques.

Notwithstanding that the numerical ranges and parameters setting forth the broad scope of the embodiments are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, *e.g.,* 5.5 to 10.

It is noted that, as used in this specification and the appended claims, the singular forms "a," "an," and "the," include plural referents unless expressly and unequivocally limited to one referent. Thus, for example, reference to "a fiber" includes one, two, three or more fibers.

### Definitions

The term "sterilization" may refer to rendering a substance incapable of reproduction, metabolism and/or growth. While this is often taken to mean total absence of living organisms, the term may be used herein to refer to a substance free from living organisms to a degree previously agreed to be acceptable. Unless otherwise indicated, the term sterilization may be used herein to also refer to methods and procedures less rigorous than sterilization, for example, disinfection, or sanitization. The sterilization indicator or biological indicator and the composition, methods, kits and apparatus described herein may be used in health care fields, scientific fields. These may be used in commercial and industrial applications where sterilization, disinfection, sanitization, may be desired, for example, sterilization, disinfection, sanitization medical, dental and/or surgical instruments and/or implants, food processing, pharmaceutical manufacturing.

The biological indicator or sterilization indicator described may be used in any sterilization process. These may include sterilization processes where the sterilization medium or sterilant may be steam, dry heat, as well as one or more gaseous sterilants, one or more liquid sterilants. The gaseous sterilants may comprise ethylene oxide, gaseous hydrogen peroxide, gaseous peracetic acid. The liquid sterilants may comprise formalin (formaldehyde gas dissolved in water and optionally containing methanol to inhibit the formation of toxic substances), glutaraldehyde, peracetic acid, liquid hydrogen peroxide.

The sterilization indicator or biological indicator may be used to examine the lethality of sterilants against any microorganism with less resistance to the sterilization process.

The term "microorganism" or "microorganisms" refers to bacteria or fungi in either the spore or vegetative state. In some embodiments, Bacillus, Clostridium, Neurospora, and/or Candida species of microorganisms are applied to the SCBI. In various embodiments, Bacillus and Clostridia species are used to monitor sterilization processes utilizing saturated steam, dry heat, gamma irradiation, and ethylene oxide. In some embodiments, microorganisms such as Geobacillus stearothermophilus and Bacillus atrophaeus monitor sterilization conditions. Geobacillus stearothermophilus is particularly useful to monitor sterilization under steam sterilization conditions. In various embodiments, microorganisms may include bacteria such as Escherichia coli, Legionella sp., Campylobacter sp., and other enteric bacteria, as well as Staphylococcus and Streptococcus species and other human pathogenic microorganisms such as Cryptosporidium.

The term "spore" refers to an asexual reproductive cell capable of developing into a new individual without fusion with another reproductive cell. The spores of the present disclosure are produced by bacteria and fungi. Bacterial spores serve largely as a resting, or dormant stage in the bacterial life cycle, serving to preserve the bacterium through periods of unfavorable conditions. Many bacterial spores are highly durable and can germinate even after years of dormancy. The bacterial spore is recognized as the most resistant form of microbial life. It is the life form of choice in all tests for determining the sterilizing efficacy of devices, implants, chemicals and processes. In one embodiment, one way of detecting whether bacteria is still present on a carrier is through enzymes. The enzyme alpha-D-glucosidase has been identified in spores of Geobacillus stearothermophilus, such as those commercially available as "ATCC 7953" from American Type Culture Collection, Rockville, MD. Bacillus atrophaeus is particularly useful to monitor conditions of gas and dry heat sterilization. The enzyme beta-D-glucosidase has been found in Bacillus atrophaeus (e.g., commercially available as "ATCC 9372" from American Type Culture Collection). In various embodiments, the spores comprise Bacillus, Clostridium, Neurospora, Candida, and, and/or Cryptosporidium. In some embodiments, the spores comprise Geobacillus stearothermophilus and/or Bacillus atrophaeus.

The term "endospore" refers to a dormant, tough, and non-reproductive structure produced by certain bacteria from the Firmicute phylum. Examples of endospores include, but are not limited to, Geobacillus stearothermophilus, Bacillus subtilis, Bacillus subtilis globigii, Bacillus atrophaeus, Clostridium sporogenes, Bacillus cereus, and Bacillus circulans. Examples of fungi include Aspergillus niger, Candida albicans, Trichophyton mentagrophytes, and Wangiella dermatitis. Examples of mycobacteria which can be utilized in the present disclosure include Mycobacterium chelonae, Mycobacterium gordonae, Mycobacterium smegmatis, and Mycobacterium terrae.

The term "vegetative bacteria" refers to a state of bacteria in which growth and reproduction occurs and where spore formation does not occur. Examples of vegetative bacteria include, but are not limited to, Aeromonas hydrophila, Enterococcus faecalis, Streptococcus faecalis, Enterococcus faecium, Streptococcus pyrogenes, Escherichia coli, Klebsiella (pneumoniae), Legionella pneumophila, Methylobacterium, Pseudomonas aeruginosa, Salmonella choleraesuis, Helicobacter pylori, Staphylococcus aureus, Staphylococcus epidermidis, and Stenotrophomonas maltophilia.

The term "glycerol" refers to a clear, colorless, viscous, liquid belonging to the alcohol family of organic compounds; molecular formula HOCH₂CHOHCH₂OH. The term "glycerin" is ordinarily applied to commercial materials containing more than 95% glycerol. In the present invention, glycerol is a humectant, a surfactant, a dispersant and/or an anti-agglomerating agent.

The term "humectant" refers to a hygroscopic substance used to keep things moist and it is the opposite of a desiccant. A humectant is often a molecule with several hydrophilic groups, most often hydroxyl groups; however, amines and carboxyl groups, sometimes esterified, can be encountered as well (its affinity to form hydrogen bonds with molecules of water, is the crucial trait). A humectant attracts and retains moisture in the air nearby via absorption, drawing the water vapor into and/or beneath the organism/object's surface. By contrast, desiccants also attract ambient moisture, but adsorb-not absorb it, by condensing the water vapor onto the surface, as a layer of film. The humectant provided on the carrier keeps the microorganism moist as it allows moisture to come in contact with the microorganism so that it does not dry out and thus the microorganism will be permeable to the sterilant and thus the sterilant can have effective and efficient kill of the microorganism on contact. In the present invention, glycerol is a humectant that is added with the inoculum of microorganisms to the carrier.

In some embodiments, other humectants may be used in addition to glycerol, such as, polyethylene glycol, propylene glycol, hexylene glycol, butylene glycol, glyceryl triacetate, neoagarobiose, sugar alcohols (sugar polyols) such as sorbitol, xylitol, maltitol, polymeric polyols such as polydextrose, quillaia, urea, aloe vera gel, MP diol, alpha hydroxy acids such as lactic acid, honey and/or lithium chloride.

The term "surfactant" refers to compounds that lower the surface tension (or interfacial tension) between two liquids or between a liquid and a solid. Surfactants may act as detergents, wetting agents, emulsifiers, foaming agents, or dispersants. Surfactants include anionic, cationic, amphoteric, non-ionic surfactants or combinations thereof. In the present invention, glycerol is a surfactant that is added with the inoculum of microorganisms to the carrier.

The term "dispersant" or a "dispersing agent" is either a non-surface active polymer or a surface-active substance added to a suspension, usually a colloid, to improve the separation of particles and to prevent settling or clumping. Typically, dispersants comprise one or more surfactants. In the preseenent invention, glycerol is a dispersant. In some embodiments, other dispersants may be used in addition to glycerol.

In some embodiments, other surfactants and/or dispersants may be in addition to glycerol, including but not limited to a polyethylene glycol, polyethoxylated castor oil, a polysorbate, a sorbitan ester, a polyoxyethylene fatty acid ester, a polyoxyethylene fatty acid ether, a polyoxyethylene alkyl ether, an ethoxylated fatty acid, long-alkyl-chain sulfonates, alkyl aryl sulfonates, dialkyl sodium sulfosuccinates, alkyl sulfates, quaternary ammonium salts, fatty alcohols such as lauryl, cetyl and stearyl alcohols; glyceryl esters such as mono-, di- and triglycerides; and fatty acid esters of fatty alcohols and other alcohols such as propylene glycol, polyethylene glycol, sorbitan, sucrose and cholesterol, polyoxyethylene glyceryl, steroidal esters, polyoxypropylene esters, and combinations thereof.

The term "anti-agglomerating agent" refers to compounds that minimize the tendency of materials (e.g., microorganisms) to stick together, clump or agglomerate. In the invention, glycerol is an anti-agglomerating agent. In some embodiments, other anti-agglomerating agents may be used in addition to glycerol, including but not limited to polyethylene glycol, talc, titanium dioxide, magnesium stearate or silicon dioxide. The anti-agglomerating agent is added with the inoculum of microorganisms to the carrier before sterilization begins.

Reference will now be made in detail to various embodiments of the present disclosure, examples of which are illustrated in the accompanying drawings. While the embodiments of the present disclosure will be described in conjunction with the illustrated embodiments, it will be understood that they are not intended to limit the disclosure to those embodiments.

The headings below are not meant to limit the disclosure in any way; embodiments under any one heading may be used in conjunction with embodiments under any other heading.

### Biological Indicator

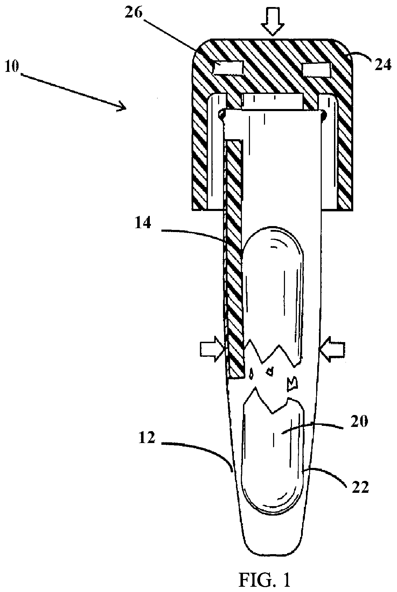

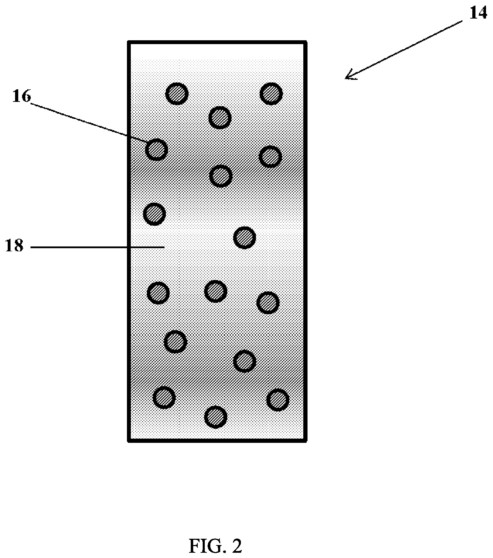

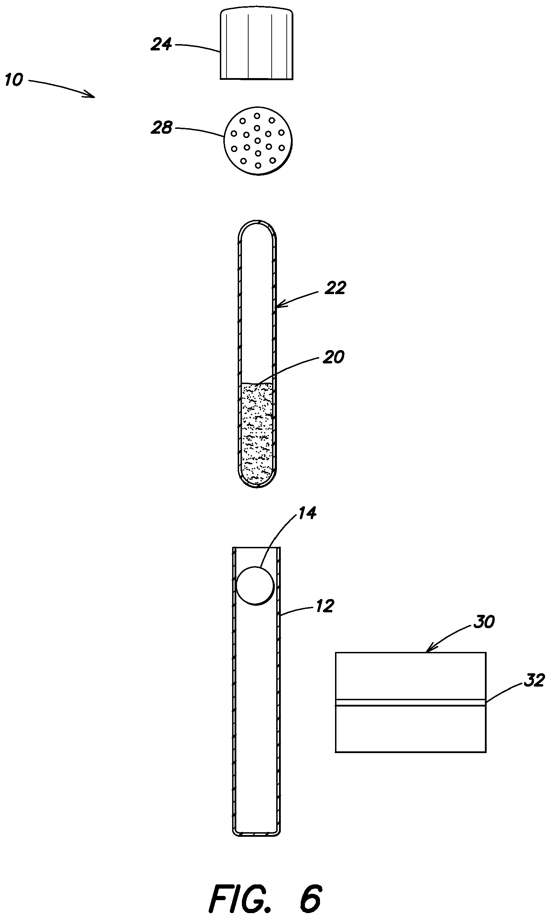

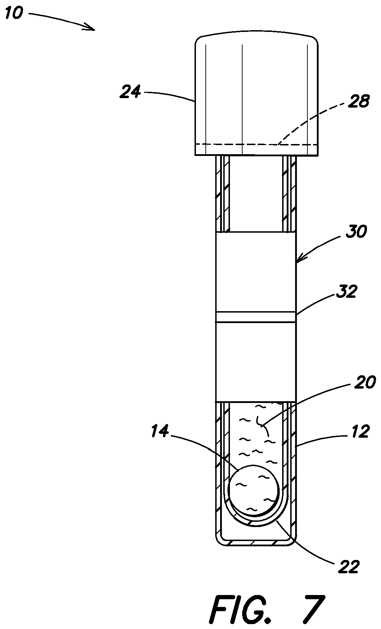

In various embodiments, a biological indicator 10 for measuring the effectiveness of a sterilization procedure is provided, as shown in FIGS. 1 to 7. Typically, the biological indicator and its components are disposable. The biological indicator comprises a container 12. The container is configured to encase or self-contain the multiple components of the biological indicator. In some embodiments, the container can be made from various materials. In some embodiments, the container comprises a glass, a metal (e.g., foil), a polymer (e.g., polycarbonate (PC), polypropylene (PP), polyphenylene (PPE), polythyene, polystyrene (PS), polyethylene, polypropylene, polyester (e.g., polyethylene terephthalate (PET)), polymethyl methacrylate (PMMA or acrylic), acrylonitrile butadiene styrene (ABS), cyclo olefin polymer (COP), cyclo olefin copolymer (COC), polysulfone (PSU), polyethersulfone (PES), polyetherimide (PEI), polybutyleneterephthalate (PBT)), a ceramic, a porcelain, or combinations thereof.

In some embodiments, the container is deformable and can be manipulated to allow a carrier containing the inoculum comprising microorganisms to contact a growth media to determine whether sterilization successfully occurred. In some embodiments, all or a portion of the container is deformable. In some embodiments, the deformable container has a modulus of elasticity from about 1 x 10⁻² to about 6 x 10⁵ dyn/cm², or 2 x 10² to about 5 x 10⁵ dyn/cm², or 5 x 10⁻² to about 5 x 10⁵ dyn/cm², or 1 x -10² to about 6 x 10⁵ dynes/cm², or 2 x 10⁴ to about 5 x 10⁵ dynes/cm², or 5 x 10⁴ to about 5 x 10⁵ dynes/cm².

The biological indicator comprises a carrier. In the embodiment shown, the carrier is shown as 14 and is disposable within the container. In some embodiments, the carrier is fixed to a section of the container. In some embodiments, the carrier is fixed to the container via a slit in the container, a ledge in the container, a ridge in the container, clips, snaps, adhesive, anchors, buttons, staples, posts, and/or fixation plates. In some embodiments, the carrier is inserted into a bifurcated section of the container. In some embodiments, the carrier is disposed in a breakable chamber of the container separated from another section of the container. In some embodiments, the carrier is freely moving in the container.

In various embodiments, the carrier can be made from a variety of materials. The carrier is configured to be inoculated with the microorganism and the humectant, anti-agglomerating agent or surfactant. The carrier will hold the microorganism and the humectant, anti-agglomerating agent or surfactant on it. The carrier will allow contact with the growth media in the biological indicator after the sterilization procedure so that any surviving microorganisms can grow and/or metabolize the nutrients in the growth media, which will cause a detectable signal that can be read by eye or machine.

In some embodiments, the carrier is made from any inorganic material such as silicon including crystalline silicon; various types of glasses including soda-lime, borosilicate glass, phosphate glass, borophosphate glass, boroaluminosilicate glass, or the like; various ceramics which can be defined as earthly raw materials in which silicon and its oxide and complex compounds known as silicates occupy a predominate portion and which have been heated to high temperatures such as structural clay products including tile and terra cotta, various porcelains, porcelain enamels, or the like; metal such as stainless steel, iron, copper; various inorganic substrates containing metalized surfaces such as those immediately set forth, or various metal oxides of groups 4 through 14 of the Periodic Table including titanium oxide, zirconium oxide, iron oxide, copper oxide, aluminum oxide, silica such as quartz, sapphire, and any combination thereof. In some embodiments of the disclosure, the carrier comprises metal oxide.

In various embodiments of the disclosure, the carrier can be made from stainless steel. The stainless steel carrier is configured to interact with the humectant, anti-agglomerating agent or surfactant, such that microorganisms inoculated onto the carrier will not clump together on the surface of the carrier, thereby causing a uniform distribution of the microorganisms. In some embodiments, an indent is defined within an end of the stainless steel carrier that is configured for engagement with the microorganisms.

In some embodiments, the carrier can be made from various organic compounds including cellulose in various forms such as paper, filter paper, chromatography paper, blotter paper cardboard, or the like. In some embodiments of the disclosure, the carrier can comprise of polymers, including, but not limited to, acrylic polymers including acrylic acid and acrylate polymers, various polyolefins such as polyethylene and polypropylene, polyvinyl alcohol polymers; polystyrene; and any combination thereof. In various embodiments of the disclosure, the carrier can be made from a combination of inorganic and organic compounds. In some embodiments of the disclosure, the carrier is made from only inorganic compounds. In some embodiments of the disclosure, the carrier is made from only organic compounds.

In some embodiments, the carrier comprises fibers. In some embodiments, the carrier comprises glass fibers. In various embodiments, the glass fibers have a diameter from about 1 to about 500 microns. In some embodiments, the glass fibers have a diameter from about 5 to about 250 microns. In some embodiments, the glass fibers have a diameter from about 50 to about 150 microns. In some embodiments, the glass fibers are about 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 405, 410, 415, 420, 425, 430, 435, 440, 445, 450, 455, 460, 465, 470, 475, 480, 485, 490, 495 and/or 500 microns.

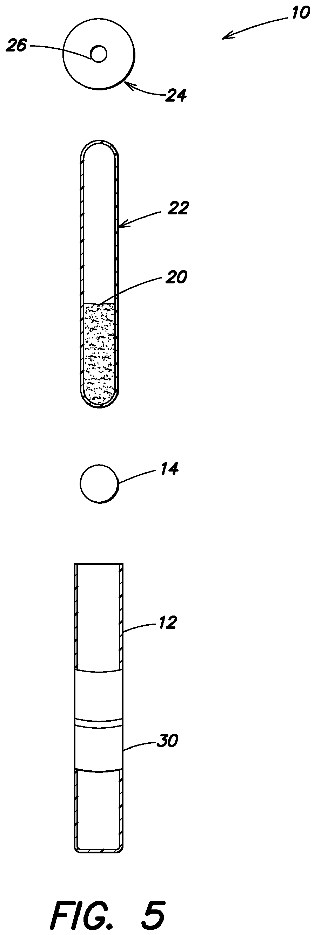

In various embodiments, the carrier is configured in various shapes such as, for example, a strip, sheet, fiber, bead, wedge, and/or ballotini® (Potters Industries, LLC, Malvern, PA). In some embodiments, the carrier is disc shaped, as shown in FIGS. 5-7.

In some embodiments, the carrier has a solidity of greater than 5% to 100%. In some embodiments, the carrier has a solidity of greater than 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100% w/w, v/w or v/v.

In some embodiments, the carrier is porous. In some embodiments, the carrier is from about 5 to about 99% porous. In various embodiments, the carrier is from about 15 to about 80% porous. In some embodiments, the carrier is from about 25 to about 65% porous. In some embodiments, the carrier is 25 to about 50% porous. In various embodiments, the carrier is 5, 10, 15, 20, 25, 30, 35, 40, 45, 50, 55, 60, 65, 70, 75, 80, 85, 90, 95, 96, 97, 98 or 99% porous. In some embodiments, the pores of the carrier are from about 1 to about 1,000 microns in size. In some embodiments, the pores of the carrier are from about 250 to about 750 microns in size. In various embodiments, the pores of the carrier are 1, 5, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990 and/or 1,000 microns.

In various embodiments, the carrier is configured to be inoculated with microorganism/microorganisms 16 and at least one of a humectant, anti-agglomerating agent or surfactant 18 disposed on the carrier. The humectant, anti-agglomerating agent or surfactant can be in solid, liquid or semi-solid form (e.g., gel).

In some embodiments of the present disclosure, the carrier is inoculated with 0.25, 0.5, 0.75, 0.78, 0.8, 0.85, 0.9, 0.95, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, 30.5, 31.0, 31.5, 32.0, 32.5, 33.0, 33.5, 34.0, 34.5, 35.0, 35.5, 36.0, 36.5, 37.0, 37.5, 38.0, 38.5, 39.0, 39.5, 40.0, 40.5, 41.0, 41.5, 42.0, 42.5, 43.0, 43.5, 44.0, 44.5, 45.0, 45.5, 46.0, 46.5, 47.0, 47.5, 48.0, 48.5, 49.0, 49.5 to about 50.0 grams out of 100g of a humectant, anti-agglomerating agent or surfactant 18 disposed on the carrier. The percentage of humectant, anti-agglomerating agent or surfactant disposed on the carrier with the microorganism can be in w/w, w/v or vv. The humectant, anti-agglomerating agent or surfactant can be in a sterile fluid, such as sterile water, distilled water, de-ionized water, water, double distilled water, NaCl (saline e.g., 0.90 saline or 0.45 saline), D5W (dextrose in 5% water), D5NS (dextrose in 5% water and normal saline), D5W/1/2NS (D5Wand ½ normal saline), alcohol (e.g., ethyl alcohol, methanol, isopropyl alcohol, etc.), nutrients or mixtures thereof. The sterile liquid (e.g., alcohol) can be volatile so it evaporates and leaves the humectant, anti-agglomerating agent or surfactant on the carrier.

In some embodiments, the carrier is inoculated with 0.5%, 1.0%, 2.0%, or 5.0% glycerol. In some embodiments of the present disclosure, the carrier is inoculated with 0.5, 1, 2, 3, 4, 5, 6, 7, 8, 9, 10, 12, 14, 16, 18, 20, 22, 24, 26, 28, 30, 32, 34, 36, 38, 40, 42, 44, 46, 48 or 50% glycerol. In some embodiments, the carrier comprises glycerol in w/w, w/v or vv. In some embodiments, the carrier is inoculate with 0.5% glycerol and 99.5% sterile fluid; 1.0% glycerol and 99.0% sterile fluid; 2.0% glycerol and 98.0% sterile fluid; or 5.0% glycerol and 95.0% sterile fluid. The sterile fluid can be sterile water, distilled water, de-ionized water, water, NaCl (saline e.g., 0.90 saline or 0.45 saline), D5W (dextrose in 5% water), D5NS (dextrose in 5% water and normal saline), D5W/1/2NS (D5Wand ½ normal saline) or combinations thereof.

In the embodiments according to the invention, the humectant, anti-agglomerating agent or surfactant comprises glycerol. In said embodiments, the carrier is inoculated with 0.5g, 1.0g, 2.0g or 5.0g out of 100g of glycerol. In some embodiments, the carrier is inoculated with 0.5%, 1.0%, 2.0%, or 5.0% glycerol. In some embodiments of the present disclosure, the carrier is inoculated with 0.5, 1, 2, 3, 4, 5, 6, 7, 8, 9, 10, 12, 14, 16, 18, 20, 22, 24, 26, 28, 30, 32, 34, 36, 38, 40, 42, 44, 46, 48 or 50% glycerol. In some embodiments, the carrier comprises glycerol in w/w, w/v or vv. In some embodiments, the carrier is inoculate with 0.5% glycerol and 99.5% sterile fluid; 1.0% glycerol and 99.0% sterile fluid; 2.0% glycerol and 98.0% sterile fluid; or 5.0% glycerol and 95.0% sterile fluid. The sterile fluid can be sterile water, distilled water, de-ionized water, water, NaCl (saline e.g., 0.90 saline or 0.45 saline), D5W (dextrose in 5% water), D5NS (dextrose in 5% water and normal saline) and D5W/1/2NS (D5Wand ½ normal saline) or a combination thereof.

In some embodiments of the disclosure, the humectant, anti-agglomerating agent or surfactant comprises polyethylene glycol. In some embodiments, the carrier is inoculated with 0.5g, 1.0g, 2.0g or 5.0g out of 100g of polyethylene glycol. In some embodiments, the carrier is inoculated with 0.5%, 1.0%, 2.0%, or 5.0% polyethylene glycol. In some embodiments, the carrier is inoculated with 0.5, 1, 2, 3, 4, 5, 6, 7, 8, 9, 10, 12, 14, 16, 18, 20, 22, 24, 26, 28, 30, 32, 34, 36, 38, 40, 42, 44, 46, 48 or 50% polyethylene glycol in a sterile fluid or dry powder. In some embodiments, the carrier comprises polyethylene glycol in w/w, w/v or vv. In some embodiments, the carrier is inoculate with 0.5% polyethylene glycol and 99.5% sterile fluid; 1.0% polyethylene glycol and 99.0% sterile fluid; 2.0% polyethylene glycol and 98.0% sterile fluid; or 5.0% polyethylene glycol and 95.0% sterile fluid. The sterile fluid can be sterile water, distilled water, de-ionized water, water, NaCl (saline e.g., 0.90 saline or 0.45 saline), D5W (dextrose in 5% water), D5NS (dextrose in 5% water and normal saline), D5W/1/2NS (D5Wand ½ normal saline) or a combination thereof.

In some embodiments of the disclosure, polyethylene glycol has a molecular weight from about 200 to about 10,000 Daltons. In various embodiments, polyethylene glycol has a molecular weight from about 1,000 to about 4,000 Daltons. In some embodiments, polyethylene glycol has a molecular weight from about 3,350 Daltons. In some embodiments, polyethylene glycol has a molecular weight from about 200 to about 600 Daltons. In various embodiments, polyethylene glycol has a molecular weight of 200, 225, 250, 275, 300, 325, 350, 375, 400, 425, 450, 475, 500, 525, 550, 575, 600, 625, 650, 675, 700, 725, 750, 775, 800, 825, 850, 875, 900, 925, 950, 975, 1,000, 1,250, 1,500, 1,750, 2,000, 2,250, 2,500, 2,750, 3,000, 3,250, 3,350, 3,500, 3,750, 4,000, 4,250, 4,500, 4,750, 5,000, 5,250, 5,500, 5,750, 6,000, 6,250, 6,500, 6,750, 7,000, 7,250, 7,500, 7,750, 8,000, 8,250, 8,500, 8,750, 9,000 9,250, 9,500, 9,750 or 10,000 Daltons.

In various embodiments of the disclosure, the carrier is inoculated with a humectant comprising glycerol and/or propylene glycol, hexylene glycol, butylene glycol, glyceryl triacetate, neoagarobiose, sorbitol, xylitol, maltitol, polymeric polyols such as polydextrose, quillaia, urea, aloe vera gel, MP diol, lactic acid, honey and/or lithium chloride. In some embodiments of the disclosure the carrier is inoculated with 0.5, 1, 2, 3, 4, 5, 6, 7, 8, 9, 10, 12, 14, 16, 18, 20, 22, 24, 26, 28, 30, 32, 34, 36, 38, 40, 42, 44, 46, 48 or 50% of a humectant. The humectant can be in liquid form, solid form or a semi-solid form (e.g., gel). In some embodiments, the carrier comprises the humectant in w/w, w/v or vv. The remainder of the humectant can be a sterile fluid, such as sterile water, distilled water, de-ionized water, water, NaCl (saline e.g., 0.90 saline or 0.45 saline), D5W (dextrose in 5% water), D5NS (dextrose in 5% water and normal saline) and D5W/1/2NS (D5Wand ½ normal saline) or mixtures thereof.

In various embodiments of the disclosure, the carrier is inoculated with an anti-agglomerating agent comprising glycerol and/or polyethylene glycol, talc, titanium dioxide, magnesium stearate and/or silicon dioxide. In some embodiments of the disclosure, the carrier is inoculated with 0.5, 1, 2, 3, 4, 5, 6, 7, 8, 9, 10, 12, 14, 16, 18, 20, 22, 24, 26, 28, 30, 32, 34, 36, 38, 40, 42, 44, 46, 48 or 50% of the anti-agglomerating agent. In some embodiments, the carrier comprises the anti-agglomerating agent in w/w, w/v or vv. The remainder can be a sterile fluid, such as sterile water, distilled water, de-ionized water, water, NaCl (saline e.g., 0.90 saline or 0.45 saline), D5W (dextrose in 5% water), D5NS (dextrose in 5% water and normal saline) and D5W/1/2NS (D5Wand ½ normal saline) or mixtures thereof. This anti-agglomerating agent reduces the microorganisms from agglomerating together and thereby increases the surface area that the sterilant has to contact the microorganism.

In various embodiments of the disclosure, the carrier is inoculated with a surfactant comprising glycerol and/or polyethoxylated castor oil, a polysorbate, a sorbitan ester, a polyoxyethylene fatty acid ester, a polyoxyethylene fatty acid ether, a polyoxyethylene alkyl ether, an ethoxylated fatty acid, long-alkyl-chain sulfonates, alkyl aryl sulfonates, dialkyl sodium sulfosuccinates, alkyl sulfates, quaternary ammonium salts, fatty alcohols, such as lauryl, cetyl and stearyl alcohols; glyceryl esters such as mono-, di- and triglycerides; and fatty acid esters of fatty alcohols and alcohols such as propylene glycol, polyethylene glycol, sorbitan, sucrose and cholesterol, polyoxyethylene glyceryl, steroidal esters and/or polyoxypropylene esters. In some embodiments of the disclosure, the carrier is inoculated with 0.5, 1, 2, 3, 4, 5, 6, 7, 8, 9, 10, 12, 14, 16, 18, 20, 22, 24, 26, 28, 30, 32, 34, 36, 38, 40, 42, 44, 46, 48 or 50% of the surfactant. In some embodiments, the carrier comprises the surfactant in w/w, w/v or vv. The remainder of the surfactant can be a sterile fluid, such as sterile water, distilled water, de-ionized water, water, NaCl (saline e.g., 0.90 saline or 0.45 saline), D5W (dextrose in 5% water), D5NS (dextrose in 5% water and normal saline) and D5W/1/2NS (D5Wand ½ normal saline) or mixtures thereof.

In some embodiments, the carrier is inoculated with a humectant, anti-agglomerating agent and/or surfactant. In some embodiments, the carrier is inoculated with only one of a humectant, anti-agglomerating agent or surfactant. In various embodiments, all or a portion of the carrier is inoculated with the humectant, anti-agglomerating agent and/or surfactant. In some embodiments, the humectant, anti-agglomerating agent and/or surfactant are evenly distributed onto the carrier. In some embodiments, the humectant, anti-agglomerating agent and/or surfactant are unevenly distributed onto the carrier at discrete positions on it.

In some embodiments, the carrier is inoculated with the microorganisms. In various embodiments, the types of microorganisms include, but are not limited to spores, endospores, bacteria, vegetative bacteria, mycobacteria and/or fungi. In some embodiments, the microorganisms include, but are not limited to Bacillus, Clostridium, Neurospora, and/or Candida species of microorganisms, which are applied to the SCBI.

In various embodiments, Bacillus and Clostridia species are used to monitor sterilization processes utilizing saturated steam, dry heat, hydrogen peroxide, peracetic acid, ethylene oxide or a combination thereof.

In some embodiments, microorganisms such as Geobacillus stearothermophilus and Bacillus atrophaeus monitor sterilization conditions. Geobacillus stearothermophilus is particularly useful to monitor sterilization under steam sterilization conditions and under hydrogen peroxide sterilization conditions. Bacillus atrophaeus is also particularly useful for ethylene oxide and dry heat sterilization. In various embodiments, microorganisms may include bacteria such as Escherichia coli, Legionella sp., Campylobacter sp., and other enteric bacteria, as well as Staphylococcus and Streptococcus species and other human pathogenic microorganisms such as Cryptosporidium.

In some embodiments, the microorganisms are spores. In various embodiments, the spores comprise Bacillus, Clostridium, Neurospora, Candida, and/or Cryptosporidium. In some embodiments, the spores comprise Geobacillus stearothermophilus and/or Bacillus atrophaeus.

In some embodiments, the microorganisms are endospores. In various embodiments, the endospores comprise Geobacillus stearothermophilus, Bacillus subtilis, Bacillus subtilis globigii, Clostridium sporogenes, Bacillus cereus, Bacillus atrophaeus and Bacillus circulans or a combination thereof.

In various embodiments, the microorganisms are fungi. In some embodiments, the fungi comprise Aspergillus niger, Candida albicans, Trichophyton mentagrophytes, Wangiella dermatitis or a combination thereof.

In some embodiments, the microorganisms are mycobacteria. In various embodiments, the mycobacteria comprise Mycobacterium chelonae, Mycobacterium gordonae, Mycobacterium smegmatis, and Mycobacterium terrae or a combination thereof.

In various embodiments, the microorganisms are vegetative bacteria. In some embodiments, the vegetative bacteria comprises Aeromonas hydrophila, Enterococcus faecalis, Streptococcus faecalis, Enterococcus faecium, Streptococcus pyrogenes, Escherichia coli, Klebsiella (pneumoniae), Legionella pneumophila, Methylobacterium, Pseudomonas aeruginosa, Salmonella choleraesuis, Helicobacter pylori, Staphylococcus aureus, Staphylococcus epidermidis, and Stenotrophomonas maltophilia or a combination thereof.

In some embodiments, the carrier is inoculated with one or a combination of the microorganisms. In various embodiments, the carrier comprises the microorganisms at a distal end of the carrier closest to the growth media in the container. In some embodiments, the carrier comprises the microorganisms disposed throughout the carrier.

In one embodiment, the concentration of the microorganisms may be in the range of from about 10¹ to about 10¹⁴ colony forming units (cfu) when disposed on the carrier. In some embodiments, the concentration of microorganisms is in the range from about 10⁴ to about 10¹⁰ cfu. In some embodiments, the concentration of microorganisms is in the range from about 10⁶ to about 10⁸ cfu. In some embodiments, the concentration of microorganisms is from about 10¹, 10², 10³ 10⁴, 10¹, 10⁶, 10⁷, 10¹, 10¹, 10¹⁰, 10¹¹, 10¹² 10¹³ or 10¹⁴ cfu.

In various embodiments, the carrier is inoculated by diluting the microorganisms in a sterile fluid (e.g., sterile water) suspension comprising the humectant, anti-agglomerating agent and/or surfactant. The fluid is then evaporated from the carrier over a particular period of time, leaving the microorganism, and the humectant, anti-agglomerating agent and/or surfactant behind on the inoculated carrier. In some embodiments, the humectant, anti-agglomerating agent and/or surfactant that remains with the microorganism on the carrier (e.g., remains after evaporation) keeps the microorganism moist as it allows moisture to come in contact with the microorganism so that it does not dry out and thus the microorganism will be permeable to the sterilant and thus the sterilant can have effective and efficient kill of the microorganism on contact.

In some embodiments, the carrier is inoculated with the microorganism, and the humectant, anti-agglomerating agent and/or surfactant via a suspension pipetted directly onto the carrier. In various embodiments, the carrier is inoculated with the microorganism, and the humectant, anti-agglomerating agent and/or surfactant by immersing or dipping the surface of the carrier into the microorganism solution, spraying, or printing a fixed volume of suspension onto the carrier on all of it or at discrete positions.

In some embodiments, the carrier further includes an ink or other suitable chemical applied to the carrier which reacts only on exposure to the sterilization process and does not undergo color transition as a result of exposure to any other sterilization agent.

In various embodiments, the biological indicator comprises growth media 20. In some embodiments, the growth media is disposed freely within the container. In some embodiments, the growth media is disposed in a vial 22. In some embodiments, the vial is crushable. The growth media is configured to be separate from the carrier until after the sterilization process has been performed. In various embodiments, after the sterilization cycle has been completed, the biological indicator is activated by squeezing the center, sides and/or the top of the deformable container, causing the crushable vial to break, thereby releasing the growth media contained within the crushable vial so that contact between the carrier containing the microorganisms and the humectant, anti-agglomerating agent and/or surfactant and the growth media occurs. In some embodiments, the crushable vial is friction fitted to the proximal end of the container where sides of the crushable vial engage inner walls of the container. The container is squeezed and/or pushed at its proximal end, causing the crushable vial to be crushed, releasing the growth media for contact with the carrier.

In some embodiments, the carrier is separate from the growth media and, after sterilization, the top part of the vial is pushed in a downward direction causing the carrier to contact the growth media so that any surviving microorganism can contact the growth media. The top part of the container can slide into the bottom part of the container to make such contact. Alternatively, the bottom part of the container can be pushed in an upward direction to cause the top part of the container having the carrier to come in contact with the growth media. Alternatively, the container can be shaken vigorously or the container inverted to cause the growth media to come in contact with the carrier.

In various embodiments, the vial and/or container can be made from various inorganic materials such as silicon including crystalline silicon; various types of glasses including soda-lime, borosilicate glass, phosphate glass, borophosphate glass, boroaluminosilicate glass, and the like; silica such as quartz, and any combination thereof. In some embodiments, the vial and/or container comprises various organic materials such as polymers, including, but not limited to, acrylic polymers including acrylic acid and acrylate polymers, various polyolefins such as polyethylene and polypropylene, polyvinyl alcohol polymers; polystyrene; and any combination thereof. In various embodiments, the vial and/or container can be made from a combination of inorganic and organic compounds.

In some embodiments, the carrier is made from only inorganic compounds. In some embodiments, the carrier is made from only organic compounds. In various embodiments, the vial comprises a stress point/points or fracture points which allow breaking of the vial. In some embodiments, the vial comprises openings such that the growth media can be released from the vial and can contact the carrier.

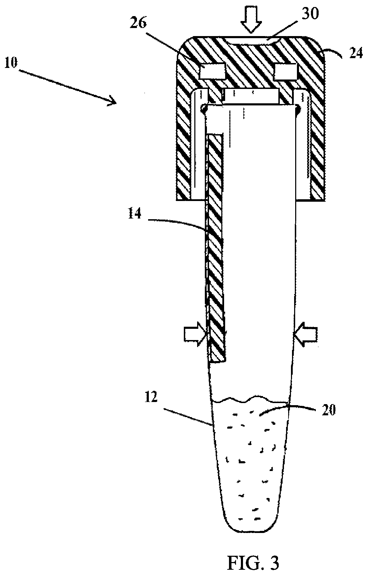

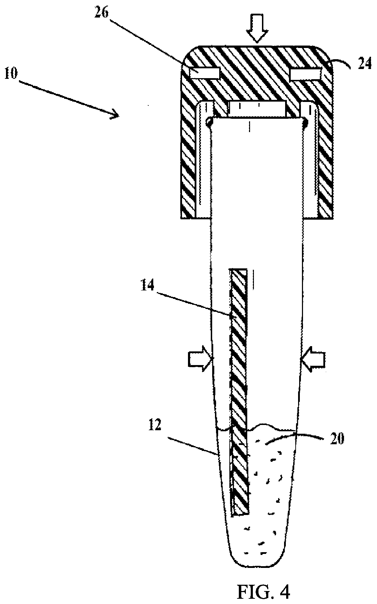

In some embodiments, the container comprises a member configured to move the carrier from a first position separated from the growth media, to a second position to contact the growth media, as shown in FIGS. 3 and 4. In some embodiments, the member comprises a barrier or a chamber. In some embodiments, the member is the crushable vial. In some embodiments, the barrier member comprises a spring system triggered by a button which actuates the movement of the carrier into the second position. In some embodiments, the member is elastic. In various embodiments, the carrier is in the first position before and during the sterilization process, as shown in FIG. 3, and the carrier is in the second position after sterilization, as shown in FIG. 4.

In some embodiments, the growth media is a liquid, solid, or a semi-solid (e.g., gel). In various embodiments, the growth media comprises soybean casein digest, pancreatic digest of casein, yeast extract, L-cysteine, tryptose sulfite cycloserine (TSC) growth media, sucrose, dextrose, dextrose tryptone, fluid thyoglycollate, nutrient broth or tryptic soy broth (TSB). In some embodiments, in steam or dry heat applications, agar-based media may be used. Agar-based media are generally semi-solid at room temperature, and upon exposure to steam or dry heat, the agar melts.

In various embodiments, the growth media comprises an indicator that undergoes a property change, which is capable of being detected and/or measured by eye or machine, in response to the growth of a particular microorganism. For example, the detector may be provided to react with a particular metabolite (e.g., an enzyme) produced by the growing microorganisms, which results in a color change, a pH change, a pH and a color change, a change in fluorescence (e.g., fluorescing or fluorescence), a change in turbidity, or the like. In some embodiments, the metabolite is selected such that relatively quick or early detection of microorganism activity is achieved.

In some embodiments, the indicator is present in an amount sufficient to provide detectable quantities of the indicator, in the presence of the metabolite, within a period of about 6 hours or less following the completion of the sterilization process. In some embodiments, the detectable quantities of the indicator in the presence of the metabolite are provided at about 24, 12, 6, 5, 4, 3, 2, 1 hour or 30 minutes. In some embodiments, the indicator may be selected based on the test microorganism being used and the metabolite of interest. In some embodiments, a metabolite is an enzyme such as alpha amylase, which is secreted in bacterium such as Bacillus subtilis, proteases, and the like. In some embodiments, indicators include, but are not limited to, biologically active molecules, fluorescent dyes, dyes, chromogenic substances, pigments, acids, bases, radiolabelled compounds, molecules that exhibit fluorescence, molecules that cease to fluoresce, and the like. In some embodiments, the indicator is a fluorescent substrate such as, for example, 4-methylumbelliferyl-α-D-glucopyroside (MUD), 4-methylumbelliferyl-β-D-galactopyronoside (MUG).

In some embodiments, the detection method may be selected based on the property of interest and may include, for example, fluorometric, visual, pH, and spectroscopic detection methods. The detection of a measurable change in an indicator property within an established period of time indicates viability of microorganisms and inadequate sterilization. The absence of a measurable change within the established period of time demonstrates that the sterilization process was lethal to the test microorganisms and, thus, adequate. In some embodiments, the detection can be determined by measuring turbidity of the growth media by eye or machine.

In some embodiments, the growth media may also comprise a substance that reduces the toxicity of the growth media toward the metabolite. In various embodiments, toxicity reducing substances include, for example, activated charcoal, bovine serum albumin, and/or a soluble starch. In some embodiments, the growth media comprises sorbitol, mannitol and/or erythritol. In various embodiments, the sorbitol, mannitol and/or erythritol comprises about 1, 2, 3, 4, 5, 6, 7, 8, 9 or 10% of the growth media.

In some embodiments, the growth media further comprises one or more pH buffers, one or more neutralizers, one or more agents for maintaining osmotic equilibrium, or a mixture of two or more thereof. In various embodiments, the pH buffers may include K₂HPO₄, KH₂PO₄, (NH₄)₂HPO₄, 2,2-Bis(hydroxylmethyl)-2,2',2"-nitrilothiethanol (Bis Tris), 1, 3-Bis[tris(hydroxymethyl)methylamino] propane (Bis-Tris Propane), 4-(2-Hydroxyethyl)piperazine-ethanesulfonic acid (HEPES), 2-Amino-2-(hydroxymethyl)-1,3-propanediol (Trizma, Tris base), N-[Tris(hydroxymethyl)methyl]glycine (Tricine), Diglycine (Gly-Gly), N,N-Bis(2-hydroxyethyl)glycine (Bicine), N-(2-Acetamido)iminodiacetic acid (ADA), N-(2-Acetamido)-2-aminoethanesulfonic acid (aces), 1,4-Piperazinediethanesulfonic acid (PIPES), β-Hydroxy-4-morpholinepropanesulfonic acid (MOPSO), N,N-Bis(2-hydroxyethyl)-2-aminoethanesulfonic acid (BES), 3-(N-Morpholino)propanesulfonic acid (MOPS), 2-[(2-Hydroxy-1,1-bis(hydroxylmethyl)ethyl)amino]ethanesulfonic acid (TES), 3-(N,N-Bis[2-hydroxyethyl]amino)-2-hydroxypropanesulfonic acid (DIPSO), 4-(N-Morpholino)butanesulfonic acid (MOBS), 2-Hydroxy-3-[tris(hydroxymethyl)methylamino]-1-propanesulfonic acid (TAPSO), 4-(2-Hydroxyethyl)piperazine-1-(2-hydroxypropanesulfonic acid hydrate (HEPPSO), Piperazine-1,4-bis(2-hydroxypropanesulfonic acid) dihydrate (POPSO), 4-(2-Hydroxyethyl)-1-piperazine propanesulfonic acid (EPPS), N-(2-Hydroxyethyl)piperazine-N'-(4-butanesulfonic acid) (HEPBS), [(2-Hydroxy-1,1-bis(hydroxymethyl)ethyl)amino]-1-propanesulfonic acid (TAPS), 2-Amino-2-methyl-1,3-propanediol (AMPD), N-tris(Hydroxymethyl)methyl-4-aminobutanesulfonic acid (TABS), N-(1,1-Dimethyl-2-hydroxyethyl)-3-amino-2-hydroxypropanesulfonic acid (AMPSO), 2-(Cyclohexylamino)ethanesulfonic acid (CHES), 3-(Cyclohexylamino)-2-hydroxyl-1-propanesulfonic acid (CAPSO), 2-Amino-2-methyl-1-propanol (AMP), 3-(Cyclohexylamino)-1-propanesulfonic acid (CAPS), 4-(Cyclohexylamino)-1-butanesulfonic acid (CABS), 2-(N-Morpholino)ethanesulfonic acid hydrate (MES), N-(2-Acetamido)-2-aminoethanesulfonic acid (ACES), and mixtures of two or more thereof.

In some embodiments, the neutralizers may include, but are not limited to sodium thioglycollate, sodium thiosulfate, catalase, sodium bisulfate, sodium bisulfite lecithin, polysorbate, polysorbate, calcium bicarbonate, and mixtures of two or more thereof. In some embodiments, the agents for maintaining osmotic equilibrium may include sodium salt, potassium salts, magnesium salts, manganese salts, calcium salts, metallic salts, sodium chloride, potassium chloride, magnesium sulfate, iron chloride, and mixtures of two or more thereof.

In various embodiments, the biological indicator comprises a cap 24 disposed at an end of the container. In some embodiments, the cap is configured to facilitate penetration of the sterilant into the biological indicator. In some embodiments, the cap comprises openings 26, such as, for example, vents and/or slits. In some embodiments, the cap is friction fitted into the end of the biological indicator. In some embodiments, the cap is configured for threaded engagement with the end of the container. In some embodiments, the cap is adhered to the end of the biological indicator via adhesive, clips, snaps and/or flanges. In some embodiments, the cap is made from various materials including, but not limited to polycarbonate, polyolefins, polyamide, polymethacrylates, polymethylpentenes, and/or polyesters.

In some embodiments, the biological indicator comprises a barrier, such as, for example, a filter 28 which is vapor-permeable and microorganism impermeable. The filter can be disposed on an inner surface of the cap or on an end of the container. The sterilant will pass through the filter to reach the inside of the container. In this way, the filter provides a controllably absorptive barrier, where the absorption of the sterilant is via chemical reaction, through which the sterilant must pass to reach the inside of the container. In some embodiments, the filter can be made from quantitative cellulose filter papers and/or polymeric filter material such as high-density polyethylene fibers. In various embodiments, the filter can be multi layered.

In some embodiments, the biological indicator comprises a sterilization marker 30. The sterilization marker will change color after a period of time, indicating whether the sterilant has properly made contact with the inside of the container. In various embodiments, the sterilization marker can be disposed on the cap of the biological indicator. In some embodiments, the sterilization marker can be disposed on the container, within the container and/or on the carrier. In various embodiments, the color change occurs via an ink or other suitable chemical applied to the sterilization marker. In various embodiments, the sterilization marker changes color based on contact with the sterilant, a pH change, a particular temperature change, and/or when moisture and/or steam contacts the sterilization marker. In some embodiments, the ink or other suitable chemical reacts only on exposure to the selected sterilant and does not undergo color transition as a result of exposure to any other sterilant or agent. In some embodiments, the sterilization marker is a chemical indicator stripe 32 disposed on an external label 30 that is fixed to an outer surface of the container.

### Methods

In various embodiments of the disclosure, a method for determining effectiveness of a sterilization procedure with a biological indicator is provided. In various embodiments, the method comprises subjecting the biological indicator to a sterilization cycle, the biological indicator comprising a deformable container having a crushable vial disposed therein comprising growth media and a carrier separated from the crushable vial but within the deformable container, wherein the carrier comprises at least one of a humectant, anti-agglomerating agent or surfactant and spores; activating the biological indicator by applying pressure to said deformable container to crush the vial so as to allow the growth media and the spores of the carrier to come into contact with each other; and incubating the biological indicator for a predetermined period of time to permit any growth of the spores surviving said sterilization cycle to grow in the growth media, wherein the growth provides a determination of the effectiveness of the sterilization procedure.

In some embodiments, the biological indicator further comprises a vented cap, as described herein, which allows a sterilant from the sterilization procedure to enter the deformable container and contact the spores on the carrier during the sterilization cycle. As the sterilant (e.g., hydrogen peroxide, peracetic acid, etc.) enters through the vented cap, it contacts the microorganisms on the carrier and should be in a sufficient quantity and time to kill all or most of the microorganisms should there be successful sterilization. In this way the object (e.g., instrument, implant, etc.) to be sterilized will be subject to the same successful sterilization procedure.

In some embodiments of the disclosure, the method comprises the biological indicator and all of its components and/or features, as described herein.

In the embodiments of the invention, the humectant, anti-agglomerating agent or surfactant comprises glycerol, as described herein. In various embodiments, the humectant, anti-agglomerating agent or surfactant may comprise glycerol and further various humectants, anti-agglomerating agents or surfactants, as described herein. In some embodiments, the spores can alternatively be any microorganism or combination of microorganisms, as described herein.

In some embodiments, the sterilization procedure comprises contacting the spores with hydrogen peroxide sterilization, steam sterilization and/or ethylene oxide sterilization. In various embodiments, the sterilization procedure comprises contacting the spores with hydrogen peroxide sterilization.

In various embodiments, the biological indicator is placed within a sterilization chamber along with objects to be sterilized. During the sterilization cycle, a portion of the sterilant (e.g., hydrogen peroxide, peracetic acid, steam, etc.) permeates through the cap's openings, infiltrating into the container where the sterilant interacts with the microorganism (e.g., spores) inoculated on the carrier.

After the sterilization cycle has been completed, the biological indicator is activated by squeezing the center, sides and/or the top of the deformable container or inverting it, which causes the crushable vial to break, thereby releasing the growth media contained within the crushable vial. The growth media will then contact the microorganisms (e.g., spores).

In some embodiments, after the sterilization cycle has been completed, the biological indicator is activated by inverting it or pushing down on its top, which causes the carrier to come in contact with the growth media, thereby releasing the growth media contained within the crushable vial. The growth media will then contact the spores.

The biological indicator is incubated for a predetermined time at an appropriate temperature. At the end of the incubation period, a detector, as described herein, is used to determine whether any spores survived the sterilization process. In various embodiments, a positive indication of spore growth is shown via a pH indicator and the pH indicator changes color in the growth media, as described herein.

In some embodiments, a log reduction indicates the effectiveness of the sterilization procedure. In some embodiments, a log reduction of at least 4 to about 12 is shown after incubation in the biological indicator. In some embodiments, a log reduction of at least 4, 5, 6, 7, 8, 9, 10, 11 or 12 is shown after incubation in the biological indicator. A log reduction of 6 means that one or less microorganisms (e.g., spores) in 1,000,000 remain following exposure to a sterilization process.

### Kits (just for illustration, not part of the invention)

In some embodiments, a kit is provided for determining the effectiveness of a sterilization procedure. In various embodiments, the kit comprises a biological indicator comprising a container having a vial disposed therein comprising growth media and a carrier separated from the vial but within the container, wherein the carrier comprises at least one of a humectant, anti-agglomerating agent or surfactant and a microorganism inoculated thereon.

In some embodiments, the humectant, anti-agglomerating agent or surfactant comprises glycerol, as described herein. In various embodiments, the humectant, anti-agglomerating agent or surfactant may comprise glycerol and/or the various humectants, anti-agglomerating agents or surfactants, as described herein. In some embodiments, the spores can alternatively be any microorganism or combination of microorganisms, as described herein.

In some embodiments, the kit comprises the biological indicator and all of its components and/or features, as described herein.

In various embodiments, the kit comprises a set of instructions on how to operate the biological indicator.

The (SCBI) of the present disclosure can be utilized by itself or in combination with a test pack to simulate the impaired access of sterilizing agent to the mass and penetration characteristics of the articles being sterilized in the sterilization chamber.

Having now generally described the invention, the same may be more readily understood through the following reference to the following examples, which are provided by way of illustration and are not intended to limit the present invention unless specified.

### EXAMPLES

When carriers comprising the spores are inoculated, spores are diluted in water and this suspension is used to inoculate the carriers. The water is evaporated from the carrier leaving the spores behind on the inoculated carrier. Should something be added to the spore/water mixture, that does not readily evaporate, it will be retained on the spore carrier in a manner similar to the spores. Experiments were conducted in which glycerol was added to the water/spore mixture. Glycerol does not readily evaporate and will remain on the spore carrier. Dependent upon the amount of glycerol added, there was a direct effect on spore resistance.

Indicators were made in which the inoculation solution contained about 0.5%, 1.0%, 2.0% and about 5.0% glycerol. Indicators with about 0.5% and about 1.0% had substantial numbers. Indicators survive in cycles described as kill cycles by the sterilizer manufacturer. Indicators with about 2.0% glycerol saw a significant reduction, and indicators with about 5.0% saw a complete elimination of surviving indicators in kill cycles.

Thus, the concentration of glycerol in the inoculum had a direct effect on the spore resistance. The concentration can be adjusted such that kill is consistently attained in kill cycles.

| Spore Disc Lot Number | Glycerol % | Spore Crop Lot Number |
|---|---|---|
| GD11 | 0.5% | 12020 |
| GD12 | 0.5% | 12020 |
| GD13-1 | 0% | 09324 |
| GD13-2 | 1% | 09324 |
| GD14 | 1% | 13165 |
| GD15 | 1% | 13165 |
| GD16A to GD16D | 1% | 13165 |
| GD17A | 1% | 13165 |
| GD17B | 2% | 13165 |
| GD17C | 5% | 13165 |
| GD18 | 2% | 13165 |
| GD19 | 1% | 13165 |
| GD20 | 1% | 13295 |
| GD21 | 1% | 13321 |
| GD22 | 2% | 13321 |
| GD23A | 1% | 13295 |
| GD23B | 5% | 13295 |
| GD23C | 2% | 13231 |
| GD23D | 2% | 14210 |
| GD23E | 5% | 14210 |
| GD23F | 2% | 14091 |
| GD23G | 5% | 14091 |
| GD23H | 2% | 14136 |
| GD23I | 5% | 14136 |
| GD24 | 5% | 13231 |

GD11 was exposed in a 100S full cycle in which the desired result was to kill of all exposed indicators. The result was 1/10 positive, indicating the indicators are too hard to kill. GD11 contained 0.5% glycerol.

GD11 was again exposed in a 100S full cycle in which the desired result was to kill of all exposed indicators. The result was 5/30 positive for the GD11 indicators. This indicates that the GD11 indicators were overly difficult to kill.

GD13-1 and GD13-2 were exposed in a 100S full cycle in which the desired result was to kill of all exposed indicators. GD13-1 and GD13-2 contain 0.0% and 1.0% glycerol respectively and the results were 7/69 and 0/60 positive for growth. This indicated that adding glycerol reduces the number of indicators that survive a cycle intended to give kill to the exposed indicators.

GD14 was exposed in 100NX Express cycle in which the desired result was to kill all of the exposed indicators. GD14 contained 1.0% glycerol because of the GD13-1 results. GD14 was 25/452 positive for growth in a cycle designed to give kill. 1% glycerol did not seem to be a complete "fix". However, GD14 also used a different spore crop which could be naturally more resistant, and/or the test number (452) was a larger load size and this too could have an effect. Because of this result, however, GD14 is considered overly resistant for the marketplace.

GD17A, GD17B, and GD17C were prepared with the same spores as GD14, with 1.0%, 2.0% and 5.0% glycerol. GD14 and GD17A are replicates of each other, both containing 1.0% glycerol. GD17A, GD17B, and GD17C were exposed in 100NX Express cycle in which the desired result was to kill of all exposed indicators. The results respectively were, 168/464 with growth, and 0/473 with growth, and 0/476 with growth. This showed an element of consistency. GD14 and GD17A, each with 1% glycerol gave 25/452 and 168/464 positive in a kill cycle. Increasing the glycerol from 1% to 2% and/or 5% showed a very clear improvement, with no indicators surviving in the kill tests at 2 and 5% glycerol.

Lots GD18 to GD22. These lots contained 1% or 2% glycerol. The results in total supported that 2% glycerol is superior to 1% glycerol in terms of desirable kill characteristics.

GD23A to GD23I. This test group was designed to compare the effects of 1%, 2% and 5% glycerol on the resistance of various spore crops.

GD23A and GD23B were made with the same spore crop lot with 1% and 5% glycerol respectively. These gave 20/20 and 0/20 positive for growth in an express cycle. This shows a clear glycerol influence.

## Claims

1. A biological indicator for measuring effectiveness of a sterilization procedure, the biological indicator comprising: a carrier having a microorganism and having from 0.5 w/w% glycerol to 5 w/w% glycerol, the carrier comprising a paper or a glass wherein the glass comprises a fiber having a diameter between 1 to 500 microns.

2. A biological indicator according to claim 1, wherein the carrier comprises (i) 0.5 w/w% glycerol and 99.5 w/w% sterile fluid; (ii) 1.0 w/w% glycerol and 99.0 w/w% sterile fluid; (iii) 2.0 w/w% glycerol and 98.0 w/w% sterile fluid; or (iv) 5.0 w/w% glycerol and 95.0 w/w% sterile fluid.

3. A biological indicator according to claim 1, wherein the microorganism comprises spores comprising Bacillus, Clostridium, Neurospora, Candida, Cryptosporidium, Geobacillus stearothermophilus and/or Bacillus atrophaeus.

4. A biological indicator of claim 1, wherein the biological indicator further comprises (i) a container comprising growth media wherein the carrier is separated from the growth media; (ii) the container comprises a member configured to move the carrier from a first position separated from the growth media to a second position to contact the growth media; or (iii) the media is disposed within a crushable vial.

5. A biological indicator according to claim 1, wherein the carrier comprises a deformable container having a modulus of elasticity from 1 x 10⁻² to 6 x 10⁵ dyn/cm².

6. A method for determining effectiveness of a sterilization procedure with a biological indicator, the method comprising: subjecting the biological indicator to a sterilization cycle, the biological indicator comprising a deformable container having a crushable vial disposed therein comprising growth media and a carrier separated from the crushable vial but within the deformable container, wherein the carrier having from 0.5 w/w% glycerol to 5 w/w% glycerol, the carrier comprising a glass wherein the glass comprising a fiber having a diameter between 1 to 500 microns and spores; activating the biological indicator by applying pressure to said deformable container to crush the vial so as to allow the growth media and the spores of the carrier to come into contact with each other; and incubating the biological indicator for a predetermined period of time to permit any growth of the spores surviving said sterilization cycle to grow in the growth media, wherein the growth provides a determination of the effectiveness of the sterilization procedure.

7. A method according to claim 6, wherein (i) the sterilization procedure comprises contacting the spores with hydrogen peroxide sterilization, steam sterilization and ethylene oxide sterilization; or (ii) the sterilization procedure comprises contacting the spores with hydrogen peroxide or peracetic acid.

8. A method according to claim 6, wherein the biological indicator further comprises a vented cap which allows a sterilant from the sterilization procedure to enter the deformable container and contact the spores on the carrier during the sterilization cycle.

9. A method according to claim 6, wherein a positive indication of spore growth is shown via a pH indicator and the pH indicator changes color in the growth media.

10. A method according to claim 6, wherein the deformable container has a modulus of elasticity from 1 x 10⁻² to 6 x 10⁵ dyn/cm².

## Patentansprüche

1. Biologischer Indikator zum Messen der Wirksamkeit einer Sterilisationsprozedur, wobei der biologische Indikator Folgendes umfasst: einen Träger mit einem Mikroorganismus und mit 0,5 Gew.-% Glycerin bis 5 Gew.-% Glycerin, wobei der Träger ein Papier oder ein Glas umfasst, wobei das Glas eine Faser mit einem Durchmesser zwischen 1 und 500 Mikrometer umfasst.

2. Biologischer Indikator nach Anspruch 1, wobei der Träger Folgendes umfasst: (i) 0,5 Gew.-% Glycerin und 99,5 Gew.-% steriles Fluid; (ii) 1,0 Gew.-% Glycerin und 99,0 Gew.-% steriles Fluid; (iii) 2,0 Gew.-% Glycerin und 98,0 Gew.-% steriles Fluid; oder (iv) 5,0 Gew.-% Glycerin und 95,0 Gew.-% steriles Fluid.

3. Biologischer Indikator nach Anspruch 1, wobei der Mikroorganismus Sporen umfasst, die Bacillus, Clostridium, Neurospora, Candida, Cryptosporidium, Geobacillus stearothermophilus und/oder Bacillus atrophaeus umfassen.

4. Biologischer Indikator nach Anspruch 1, wobei der biologische Indikator ferner (i) einen Behälter mit Wachstumsmedium umfasst, wobei der Träger von dem Wachstumsmedium getrennt ist; (ii) der Behälter ein Element umfasst, das dazu konfiguriert ist, den Träger aus einer ersten Position, die von dem Wachstumsmedium getrennt ist, in eine zweite Position zu bewegen, um mit dem Wachstumsmedium in Kontakt zu treten; oder (iii) das Medium in einem zerdrückbaren Fläschchen angeordnet ist.

5. Biologischer Indikator nach Anspruch 1, wobei der Träger einen verformbaren Behälter mit einem Elastizitätsmodul von 1 x 10⁻² bis 6 x 10⁵ dyn/cm² umfasst.

6. Verfahren zum Bestimmen der Wirksamkeit einer Sterilisationsprozedur mit einem biologischen Indikator, wobei das Verfahren Folgendes umfasst: Unterziehen des biologischen Indikators einem Sterilisationszyklus, wobei der biologische Indikator einen verformbaren Behälter mit einem darin angeordneten zerdrückbaren Fläschchen umfasst, umfassend das Wachstumsmedien und einen Träger, der sich von dem zerdrückbaren Fläschchen getrennt, jedoch innerhalb des verformbaren Behälters befindet, wobei der Träger von 0,5 Gew.- % Glycerin bis 5 Gew.-% Glycerin aufweist, wobei der Träger ein Glas umfasst, wobei das Glas eine Faser mit einem Durchmesser zwischen 1 und 500 Mikrometer und Sporen umfasst; Aktivieren des biologischen Indikators durch Ausüben von Druck auf den verformbaren Behälter, um das Fläschchen zu zerdrücken, damit das Wachstumsmedium und die Sporen des Trägers miteinander in Kontakt kommen können; und Inkubieren des biologischen Indikators für einen vorbestimmten Zeitraum, um jegliches Wachstum der Sporen, die den Sterilisationszyklus überleben, in den Wachstumsmedien wachsen zu lassen, wobei das Wachstum eine Bestimmung der Wirksamkeit der Sterilisationsprozedur bereitstellt.

7. Verfahren nach Anspruch 6, wobei (i) die Sterilisationsprozedur die Sterilisation durch Inkontaktbringen der Sporen mit Wasserstoffperoxid, Dampf und Ethylenoxid umfasst; oder (ii) die Sterilisationsprozedur das Inkontaktbringen der Sporen mit Wasserstoffperoxid oder Peressigsäure umfasst.

8. Verfahren nach Anspruch 6, wobei der biologische Indikator ferner eine Entlüftungskappe umfasst, die es einem Sterilisationsmittel aus der Sterilisationsprozedur ermöglicht, in den verformbaren Behälter einzutreten und während der Sterilisationszyklus mit den Sporen auf dem Träger in Kontakt zu treten.

9. Verfahren nach Anspruch 6, wobei ein positiver Hinweis auf Sporenwachstum über einen pH-Indikator gezeigt wird und der pH-Indikator die Farbe in dem Wachstumsmedium ändert.

10. Verfahren nach Anspruch 6, wobei der verformbare Behälter einen Elastizitätsmodul von 1 x 10⁻² bis 6 x 10⁵ dyn/cm² aufweist.

## Revendications

1. Indicateur biologique pour mesurer l'efficacité d'une procédure de stérilisation, l'indicateur biologique comprenant: un support ayant un micro-organisme et ayant de 0,5 % p/p de glycérol à 5 % p/p de glycérol, le support comprenant un papier ou un verre, dans lequel le verre comprend une fibre ayant un diamètre compris entre 1 et 500 microns.

2. Indicateur biologique selon la revendication 1, dans lequel le support comprend (i) 0,5 % p/p de glycérol et 99,5 % p/p de liquide stérile; (ii) 1,0 % p/p de glycérol et 99,0 % p/p de liquide stérile; (iii) 2,0 % p/p de glycérol et 98,0 % p/p de liquide stérile; ou (iv) 5,0 % p/p de glycérol et 95,0 % p/p de liquide stérile.

3. Indicateur biologique selon la revendication 1, dans lequel le micro-organisme comprend des spores comprenant Bacillus, Clostridium, Neurospora, Candida, Cryptosporidium, Geobacillus stearothermophilus et/ou Bacillus atrophaeus.

4. Indicateur biologique selon la revendication 1, dans lequel l'indicateur biologique comprend en outre (i) un récipient comprenant un milieu de croissance, dans lequel le support est séparé du milieu de croissance; (ii) le récipient comprend un élément configuré pour déplacer le support d'une première position séparée du milieu de croissance à une seconde position pour entrer en contact avec le milieu de croissance; ou (iii) le milieu est disposé à l'intérieur d'un flacon écrasable.

5. Indicateur biologique selon la revendication 1, dans lequel le support comprend un récipient déformable ayant un module d'élasticité de 1 x 10⁻² à 6 x 10⁵ dyn/cm².

6. Procédé pour déterminer l'efficacité d'une procédure de stérilisation avec un indicateur biologique, le procédé comprenant: la soumission de l'indicateur biologique à un cycle de stérilisation, l'indicateur biologique comprenant un récipient déformable dans lequel est disposé un flacon écrasable comprenant un milieu de croissance et un support séparé du flacon écrasable mais à l'intérieur du récipient déformable, dans lequel le support a 0,5 % p/p de glycérol à 5 % p/p de glycérol, le support comprenant un verre, dans lequel le verre comprend une fibre ayant un diamètre compris entre 1 et 500 microns et des spores; l'activation de l'indicateur biologique en appliquant une pression sur ledit récipient déformable pour écraser le flacon de manière à permettre au milieu de croissance et aux spores du support d'entrer en contact les uns avec les autres; et l'incubation de l'indicateur biologique pendant une période de temps prédéterminée pour permettre à toute croissance des spores survivant audit cycle de stérilisation de se développer dans le milieu de croissance, dans lequel la croissance fournit une détermination de l'efficacité de la procédure de stérilisation.

7. Procédé selon la revendication 6, dans lequel (i) la procédure de stérilisation comprend la mise en contact des spores avec une stérilisation au peroxyde d'hydrogène, une stérilisation à la vapeur et une stérilisation à l'oxyde d'éthylène; ou (ii) la procédure de stérilisation comprend la mise en contact des spores avec du peroxyde d'hydrogène ou de l'acide peracétique.

8. Procédé selon la revendication 6, dans lequel l'indicateur biologique comprend en outre un capuchon ventilé qui permet à un stérilisant issu de la procédure de stérilisation d'entrer dans le récipient déformable et de venir en contact avec les spores sur le support pendant le cycle de stérilisation.

9. Procédé selon la revendication 6, dans lequel une indication positive de la croissance des spores est représentée via un indicateur de pH et l'indicateur de pH change de couleur dans le milieu de croissance.

10. Procédé selon la revendication 6, dans lequel le récipient déformable a un module d'élasticité de 1 x 10⁻² à 6 x 10⁵ dyn/cm².
